Europäisches Patentamt

European Patent Office

Office européen des brevets

(19)

(11) Veröffentlichungsnummer: **0 363 601**
**A2**

(12) **EUROPÄISCHE PATENTANMELDUNG**

(21) Anmeldenummer: **89114865.2**

(22) Anmeldetag: **11.08.89**

(51) Int. Cl.⁵: **C07H 15/04 , C07H 11/00**

(30) Priorität: **13.10.88 DE 3834911**

(43) Veröffentlichungstag der Anmeldung:
**18.04.90 Patentblatt 90/16**

(84) Benannte Vertragsstaaten:
**DE FR GB IT**

(71) Anmelder: **HÜLS AKTIENGESELLSCHAFT**
**Patentabteilung / PB 15 - Postfach 13 20**
**D-4370 Marl 1(DE)**

(72) Erfinder: **Ripke, Norbert, Dr.**
**Hellweg 28**
**D-4358 Haltern(DE)**

(54) **Verfahren zur Sulfatierung von Alkyloligoglycosiden.**

(57) Übliche Sulfatierungen werden lösemittelfrei durchgeführt. Bekannt ist ferner, daß man Alkylglycoside mit einem Schwefeltrioxid-Amin-Komplex in Dimethylformamid sulfatieren kann.

Erfindungsgemäß werden Alkyloligoglycoside mit üblichen Sulfatierungsmitteln in niedrigsiedenden Lösemitteln bei geringer Nebenproduktbildung schonend sulfatiert.

Herstellung sulfatierter Alkyloligoglycoside.

EP 0 363 601 A2

## Verfahren zur Sulfatierung von Alkyloligoglycosiden

Die Erfindung betrifft ein Verfahren zur Sulfatierung von Alkyloligoglycosiden mit Alkylresten mit 8 bis 22 C-Atomen und einem mittleren Oligomerisationsgrad von 1 bis 5 mit Hilfe von üblichen Sulfatierungsmitteln.

Sulfatierte Alkyloligoglycoside werden als Schaumbildner und Dispersionsmittel in Spül-, Wasch- und Reinigungsmitteln verwendet. Es sind anionische Tenside, die in Kosmetika und bei der tertiären Erdölförderung eingesetzt werden.

Nach US-PS 1 951 784 können Schwefelsäureester von Alkyloligoglycosiden durch simultane Reaktion von Sacchariden oder Stärke mit Schwefelsäure und einem Alkohol hergestellt werden. Nach diesem Verfahren entstehen in erheblichem Maße auch Schwefelsäureester der eingesetzten Alkohole, wodurch die Ausbeute an gewünschtem Produkt deutlich gemindert wird.

Geht man aber von Alkyloligoglycosiden aus, die einen Schmelzpunkt im Bereich von 150 °C aufweisen, so sind Sulfatierungstemperaturen von über 100 °C erforderlich. Bei diesen Temperaturen erfolgt als Nebenreaktion eine Acetalspaltung, worauf die frei gewordenen Alkohole ebenfalls zu Schwefelsäureestern umgesetzt werden. Außerdem wird dabei Glucose freigesetzt, die unter diesen Bedingungen zu Kohle verascht.

Nach EP-A-280 715 können Alkylglycoside mit Hilfe eines Schwefeltrioxid-Trimethylamin-Komplexes in Dimethylformamid unter milden Bedingungen in 19 bis 24 Stunden sulfatiert werden. Dieses Verfahren ist durch die Verwendung des speziellen Reagenz aufwendig. Außerdem ist der hohe Siedepunkt bei der destillativen Rückgewinnung des Lösemittels von Nachteil.

Aufgabe der vorliegenden Erfindung ist es, ein vereinfachtes Verfahren zur schonenden Sulfatierung von Alkyloligoglycosiden bereitzustellen.

Die Aufgabe wird erfindungsgemäß dadurch gelöst, daß man die Sulfatierung unter Verwendung von üblichen Sulfatierungsmitteln in inerten organischen Lösemitteln mit einem Siedepunkt (unter Normalbedingungen) von 30 bis 100 °C durchführt.

Übliche Sulfatierungsmittel sind dabei Chlorsulfonsäure, Schwefeltrioxid, 70- bis 100%ige Schwefelsäure oder Schwefeltrioxid enthaltende Schwefelsäure, wobei auf 100 Mole Schwefelsäure bis zu 70 Mole Schwefeltrioxid kommen können.

Für die Sulfatierung werden Alkyloligoglycoside eingesetzt, die verzweigte oder unverzweigte Alkylreste mit 8 bis 22 C-Atomen enthalten. Vorzugsweise werden Alkyloligoglycoside mit Alkylresten mit 10 bis 16 C-Atomen eingesetzt.

Die Alkyloligoglycoside weisen einen mittleren Oligomerisationsgrad von 1 bis 5 auf. Im Sinne der vorliegenden Erfindung werden danach auch Alkylglycoside (Oligomerisationsgrad 1) als Alkyloligoglycoside bezeichnet.

Die den Alkyloligoglycosiden zugrunde liegenden Saccharide sind Hexosen, die in der furanosen oder in der pyranosen Form vorliegen können. Geeignete Hexosen sind beispielsweise Glucose, Galactose, Maltose, Mannose oder Fructose. Vorzugsweise verwendet man Alkyloligoglucoside.

Die Alkyloligoglycoside werden als 5- bis 95%ige Lösung in einem niedrigsiedenden organischen Lösemittel für die Sulfatierung eingesetzt. Die Konzentration beträgt vorzugsweise 20 bis 60 %.

Geeignete Lösemittel sind Halogenkohlenwasserstoffe, wie beispielsweise Methylenchlorid und Chloroform, Ether, wie Diethylether, Diisopropylether und Tetrahydrofuran, Benzol sowie Schwefelkohlenstoff.

Vorzugsweise werden Lösemittel mit einem Siedepunkt im Bereich von 35 bis 70 °C verwendet. Insbesondere setzt man Halogenkohlenwasserstoffe als Lösemittel ein. Amine, Alkohole und Aldehyde sind dagegen als Lösemittel ungeeignet, da sie gegen Schwefeltrioxid und Chlorsulfonsäure nicht inert sind.

Die Sulfatierungstemperatur liegt bei -20 bis +100 °C. Dabei wird im allgemeinen eine Temperatur gewählt, die unterhalb des Siedepunktes des verwendeten Lösemittels liegt. Die Temperatur liegt vorzugsweise bei 0 bis 60 °C, wobei Raumtemperatur ganz besonders bevorzugt wird.

Die Reaktionsgeschwindigkeit ist auch unter diesen milden Bedingungen sehr hoch.

Die Sulfatierung kann in üblichen Rührkesseln durchgeführt werden. Mit Vorteilen werden hier jedoch Reaktoren verwendet, in denen die Reaktion in einer dünnen Schicht erfolgt. Beispielsweise können schonend arbeitende Fallfilmreaktoren eingesetzt werden, wie sie unter anderem in der Zeitschrift "Seifen-Öle-Fette-Wachse", Nr. 13, (1973), Seiten 360 bis 365, beschrieben werden. Derartige Reaktoren bestehen im wesentlichen aus einem temperierten Zylinder, an dessen Kopfende eine Düse eingebaut ist. Zur Sulfatierung fließt ein dünner Film Alkyloligoglycosid-Lösung an der Zylinderinnenwand herunter, während das Sulfatierungsmittel durch die Düse auf die Lösung gesprüht wird.

Der Fallfilmreaktor erlaubt eine gleichmäßige Sulfatierung bei sehr kurzen Kontaktzeiten.

Nach dem erfindungsgemäßen Verfahren können sämtliche Hydroxylgruppen der Alkyloligoglycoside mit Schwefelsäure verestert werden.

Die Sulfatierungsbedingungen sind so milde, daß die Acetalbindungen nicht hydrolysiert werden. Man erhält deshalb auch keine langkettigen Alkohole oder Schwefelsäureester langkettiger Alkohole als Nebenprodukt.

Die Lösung bleibt stippenfrei. Der Umsatz, bezogen auf das im Unterschuß zugesetzte Sulfatierungsmittel, ist praktisch quantitativ.

Dadurch wird eine definierte Sulfatierung ermöglicht.

Mit üblichen Sulfatierungsmitteln erhält man Produkte, die aminfrei und deshalb gesundheitlich unbedenklich sind.

Das Sulfatierungsverfahren kann kontinuierlich und diskontinuierlich ausgeführt werden.

Nach dem erfindungsgemäßen Verfahren wird das Alkyloligoglycosid im allgemeinen in dem inerten Lösemittel vorgelegt und das Sulfatierungsmittel zugegeben. Das Sulfatierungsmittel kann als Flüssigkeit zugetropft werden. Schwefeltrioxid wird dagegen vorzugsweise mit Hilfe eines Inertgases in die Lösung eingeleitet oder über eine Düse auf die Lösung gesprüht. Schwefelsäure, Chlorsulfonsäure und dergleichen können ebenfalls mittels einer Düse versprüht werden.

Nach beendeter Reaktion wird neutralisiert, worauf das Lösemittel destilliert wird. Als Rückstand erhält man dabei Salze der sulfatierten Alkyloligoglycoside. Durch Ansäuern können daraus die freien Schwefelsäurehalbester der Alkyloligoglycoside erhalten werden.

Die folgenden Beispiele sollen die Erfindung verdeutlichen. Konzentrationen werden dabei, wenn nicht anders angegeben, in Gewichtsprozent berechnet.

Beispiel 1

In einem 3-l-Dreihalskolben, der mit Rührer, Thermometer, Tropftrichter und Rückflußkühler ausgestattet ist, werden 1,5 kg 20%ige Lösung von Dodecyloligoglucosid (mittlerer Oligomerisationsgrad: 1,5) in Chloroform vorgelegt. Bei 20 °C werden in 1 Stunde 203 g Chlorsulfonsäure ($\hat{=}$ 0,5 eq/OH) zugetropft. Anschließend wird noch 1 Stunde nachgerührt, worauf mit 10%iger wäßriger Natronlauge neutralisiert wird. Nach Destillation des Lösemittels erhält man als Rückstand
1,5 kg sulfatiertes Dodecyloligoglucosid
SZ gem.: 45 mg KOH/g; SZ theor.: 55mg KOH/g
(SZ = Säurezahl)

Beispiel 2

In der Apparatur von Beispiel 1 werden 1,5 kg 20%ige Lösung von Tetradecyloligoglucosid (mittlerer Oligomerisationsgrad: 1,5) in Methylenchlorid vorgelegt. Bei 0 °C werden dann in 1,5 Stunden 18 g 95%ige Schwefelsäure ($\hat{=}$ 0,05 eq/OH) zugetropft. Es wird 30 Minuten nachgerührt, worauf man mit 10%iger wäßriger Kalilauge neutralisiert. Anschließend wird Methylenchlorid destilliert. Als Rückstand erhält man
1,5 kg sulfatiertes Tetradecyloligoglucosid
SZ gem.: 4,8 mg KOH/g; SZ theor.: 5,5 mg KOH/g

Beispiel 3

In einem 4-l-Rührtopf, der mit Frittenboden, Rührer, Thermometer und Rückflußkühler ausgestattet ist, werden 1,5 kg 20%ige Lösung von Dodecyloligoglucosid (mittlerer Oligomerisationsgrad: 2,5) in Chloroform vorgelegt. Bei 20 °C werden in 1,5 Stunden 86,4 g Schwefeltrioxid ($\hat{=}$ 0,3 eq/OH), verdünnt mit Stickstoff, durch den Frittenboden von unten in die Reaktionsmischung geleitet. Das Ende der Reaktion wird dünnschichtchromatographisch bestimmt.

Anschließend wird mit 10%iger wäßrige Kalilauge neutralisiert, worauf das Lösemittel abdestilliert wird. Als Rückstand erhält man
1,5 kg sulfatiertes Dodecyloligoglucosid
SZ gem.: 28 mg KOH/g; SZ theor.: 33 mg KOH/g

Beispiel 4

Durch einen Fallfilmreaktor (Länge: 1,2 m, Innendurchmesser: 25 mm) werden bei 20 °C 1,5 kg 20%ige Lösung von Tetradecyloligoglucosid (mittlerer Oligomerisationsgrad: 2) in Chloroform gepumpt. Gleichzeitig werden mittels einer am Kopfende angebrachten Düse 28,4 g Schwefeltrioxid, verdünnt mit Stickstoff (Molverhältnis $SO_3/N_2$ = 1 : 7,5), versprüht.

Die abfließende Lösung wird direkt mit 10%iger wäßriger Kalilauge neutralisiert, und die Lösemittel werden destilliert. Als Rückstand erhält man
1,5 kg sulfatiertes Tetradecyloligoglucosid
SZ gem.: 64,9 KOH/g; SZ theor.: 66,4 mg KOH/g

Ansprüche

1. Verfahren zur Sulfatierung von Alkyloligoglycosiden mit einem Alkylrest mit 8 bis 22 C-Atomen und einem mittleren Oligomerisationsgrad von 1 bis 5 mit Hilfe von üblichen Sulfatierungsmitteln, dadurch gekennzeichnet, daß man die Sulfatierung in einem organischen Lösemittel mit einem Siedepunkt von 30 bis 100 °C durchführt.

2. Verfahren nach Anspruch 1,

dadurch gekennzeichnet,
daß die Reaktion in einem Lösemittel mit einem Siedepunkt von 35 bis 70 °C durchgeführt wird.

3. Verfahren nach Anspruch 1,
dadurch gekennzeichnet,
daß man die Reaktion in einem chlorierten Kohlenwasserstoff durchführt.

4. Verfahren nach Anspruch 1,
dadurch gekennzeichnet,
daß man 5- bis 95%ige Lösungen der Alkyloligoglycoside für die Sulfatierung einsetzt.

5. Verfahren nach Anspruch 4,
dadurch gekennzeichnet,
daß man 20- bis 60%ige Lösungen einsetzt.

6. Verfahren nach Anspruch 1,
dadurch gekennzeichnet,
daß man Alkyloligoglycoside mit einem Alkylrest mit 10 bis 16 C-Atomen sulfatiert.

7. Verfahren nach Anspruch 1,
dadurch gekennzeichnet,
daß man die Sulfatierung bei -20 bis +100 °C, vorzugsweise bei 0 bis 60 °C durchführt.

8. Verfahren nach Anspruch 1,
dadurch gekennzeichnet,
daß man die Reaktion in einem Fallfilmreaktor durchführt.